# EUROPÄISCHE PATENTSCHRIFT

(11) **EP 2 641 539 B1**
(45) Veröffentlichungstag und Bekanntmachung des Hinweises auf die Patenterteilung: **08.12.2021**
(21) Anmeldenummer: 13159717.1
(22) Anmeldetag: 18.03.2013
(51) Int. Cl.: A61B 5/107, A41H 1/02, A43D 1/08, G01B 11/24

(54) **Verfahren zum Bestimmen der Abmessungen eines Fußes**
Method for determining the dimensions of a foot
Procédé destiné à la détermination des dimensions d'un pied

(30) Priorität: 21.03.2012 DE 102012204537
(43) Veröffentlichungstag der Anmeldung: 25.09.2013
(73) Patentinhaber: OneFID GmbH, 96215 Lichtenfels (DE)
(72) Erfinder: Harmes, Thomas, 47807 Krefeld (DE); Köhler, Dominic, 47807 Krefeld (DE); Lessel, Dominik, 47807 Krefeld (DE)
(74) Vertreter: Hafner & Kohl PartmbB

(56) Entgegenhaltungen:
- EP-A1- 1 044 648
- EP-A2- 0 324 561
- WO-A1-2008/145952
- WO-A1-2011/161285
- WO-A1-2013/026798
- WO-A2-2011/142655
- AU-A1- 2004 202 110
- US-A1- 2002 126 295
- US-A1- 2010 030 578
- US-A1- 2010 319 100
- US-A1- 2011 055 054
- CHAO-YANG YANG ET AL: "Application of Virtual Reality Technology in Online Fashion Store Development", COMPUTATIONAL INTELLIGENCE AND SOFTWARE ENGINEERING, 2009. CISE 2009. INTERNATIONAL CONFERENCE ON, IEEE, PISCATAWAY, NJ, USA, 11. Dezember 2009 (2009-12-11), Seiten 1-6, XP031588751, ISBN: 978-1-4244-4507-3

## Beschreibung

Die Erfindung betrifft ein Verfahren zum Bestimmen der Abmessungen mindestens eines Fußes eines Nutzers.

Möchte ein Nutzer beispielsweise über den Versandhandel ein Kleidungsstück bestellen, so kann er dieses vor dem Bestellen nicht anprobieren. Üblicherweise bestellt daher ein Nutzer ein Kleidungsstück, indem er z.B. seine Konfektionsgröße oder seine Schuhgröße angibt. Alternativ kann ein Nutzer beispielsweise unter Verwendung eines Maßbandes die Abmessungen bestimmter Körperteile ermitteln und z.B. über standardisierte Maßtabellen seine Konfektionsgröße bestimmen und diese bei der Bestellung übermitteln, so dass ihm ein Kleidungsstück geliefert wird, das der ermittelten Konfektionsgröße entspricht.

Das Bestellen von Kleidungsstücken, beispielsweise von Oberbekleidung oder Schuhen, nach standardisierten Konfektions- oder Schuhgrößen hat den Nachteil, dass Kleidungsstücke verschiedener Hersteller in der gleichen Größe teils erhebliche Unterschiede in den Abmessungen aufweisen können. Somit kann nicht gewährleistet werden, dass das bestellte Kleidungsstück mit den Abmessungen des Körperteils des Nutzers übereinstimmt und somit eine gute Passform aufweist. Ein manuelles Abmessen eines Körperteils durch den Nutzer selbst kann zwar unter Umständen genauere Ergebnisse liefern, ist jedoch mit einem hohen Aufwand verbunden und lässt daher viele Nutzer vor einer Bestellung von Kleidung im Versandhandel zurückschrecken. Ferner ist das manuelle Abmessen von Körperteilen durch den Nutzer selbst fehleranfällig, so dass möglicherweise durch den Nutzer falsche Größenangaben ermittelt und der Bestellung zugrunde gelegt werden.

Das nachveröffentlichte Dokument WO 2013/026798 A1 offenbart ein Prinzip zur Auswahlen von Schuhen geeigneter Größe.

Das Dokument WO 2011/161285 A1 offenbart ein Prinzip zur Ermittlung der Abmessungen eines Fußes.

Das Dokument US 2010/0030578 A1 offenbart ein Prinzip zur Zusammenarbeit in Online-Gemeinschaften.

Aufgabe der Erfindung ist es, ein Verfahren bereitzustellen, mit dem einfach und genau die Abmessungen eines Fußes eines Nutzers bestimmt werden können.

Die Lösung der Aufgabe erfolgt erfindungsgemäß durch die Merkmale des Anspruchs 1.

Beim erfindungsgemäßen Verfahren werden die Abmessungen mindestens eines Fußes eines Nutzers mit den im Folgenden beschriebenen Schritten bestimmt.

Zunächst erfolgt ein Fotografieren des Fußes oder des auf einer Darstellungsfläche aufgezeichneten Umrisses mindestens eines Fußes und eines Referenzobjekts mit bekannten Abmessungen. Es können auch mehrere Füße, deren Abmessungen bestimmt werden sollen, gleichzeitig fotografiert werden. Wesentlich ist, dass der Fuß, dessen Abmessungen bestimmt werden sollen, oder der Umriss sowie das Referenzobjekt auf demselben Foto abgebildet sind. Die Fotografie kann durch den Nutzer selbst, durch eine zweite Person oder mit Hilfe eines Stativs und Selbstauslöser aufgenommen werden. Die Fotografie erfolgt mit einer Digitalkamera eines Mobilfunkendgeräts, beispielsweise eines Smartphones. Bei der Verwendung der Digitalkamera eines Smartphones bietet sich der Vorteil, dass weitere Verfahrensschritte des erfindungsgemäßen Verfahrens durch eine Software ausgeführt werden, die als Applikation auf dem Smartphone bzw. einem Server installiert ist.

Die Abmessungen des Fußes werden anschließend anhand der Fotografie basierend auf den bekannten Abmessungen des Referenzobjekts errechnet.

Als Abmessungen können beispielsweise die maximale Länge und die maximale Breite eines Fußes, von der Blickrichtung der verwendeten Kamera aus betrachtet, errechnet werden. Alternativ können auch mehr Informationen hinsichtlich der Abmessungen des Fußes errechnet werden. Insbesondere ist es möglich, die Länge und/ oder Breite eines Fußes an verschiedenen Stellen des Fußes zu bestimmen oder sogar den genauen Verlauf des gesamten Umrisses des Fußes zu bestimmen. Dies kann beispielsweise sinnvoll sein für Kleidungsstücke, bei denen eine genaue Passform sehr wichtig ist, z.B. für Schuhe oder andere Bekleidung. Durch das erfindungsgemäße Verfahren kann beispielsweise herausgefunden werden, dass ein Nutzer an einer bestimmten Stelle einen besonders breiten oder besonders schmalen Fuß hat und somit bestimmte Schuhmodelle besonders geeignet sind, während andere Schuhmodelle weniger geeignet sind.

Entsprechend den bestimmten Abmessungen des fotografierten Fußes können dem Nutzer z.B. bestimmte Vorschläge hinsichtlich eines passenden Kleidungsstückes unterbreitet werden. Weitere Details hierzu werden im weiteren Verlauf der vorliegenden Anmeldung beschrieben.

Das erfindungsgemäße Verfahren bietet somit eine einfache und zuverlässige Möglichkeit, die Abmessungen eines Fußes eines Nutzers zu bestimmen, ohne dass dieser aufwändige Messungen durchführen muss. Auch die Gefahr einer Falschmessung kann durch das erfindungsgemäße Verfahren minimiert werden. Das erfindungsgemäße Verfahren vereinfacht somit erheblich die Bestellung von Kleidungsstücken über den Versandhandel sowie die Größenfindung im stationären Einzelhandel.

Wesentliches Merkmal des erfindungsgemäßen Verfahrens ist die Verwendung eines Referenzobjekts, dessen Abmessungen bekannt sind. Hierbei handelt es sich um weißes Blatt mit einem normierten Format.

Es ist bevorzugt, dass das Referenzobjekt und der fotografierte Fuß oder die Darstellungsfläche mit dem Umriss des Fußes zum Zeitpunkt des Fotografierens in einer gemeinsamen Ebene angeordnet sind. Alternativ können sie in zwei Ebenen angeordnet sein, deren Abstand und Winkel zueinander bekannt sind. Unter Berücksichtigung der bekannten Abmessungen des Referenzobjekts und der bekannten räumlichen Lage des Referenzobjekts gegenüber dem fotografierten Fuß oder der fotografierten Darstellungsfläche ist es möglich, die Abmessungen des Fußes präzise zu berechnen.

Es ist bevorzugt, dass das Referenzobjekt und die Darstellungsfläche identisch sind. Erfindungsgemäß handelt es sich hierbei um ein Blatt mit genormter Größe. Besonders bevorzugt kann ein DIN A4-Blatt verwendet werden, da seine Abmessungen sehr gut dazu geeignet sind, z.B. den Fuß eines Erwachsenen hierauf zu platzieren. Ein DIN A4-Blatt mit weißer Farbe bietet den Vorteil eines hohen Kontrastes gegenüber dem fotografierten Fuß eines Nutzers. Hierzu kann der Nutzer beispielsweise eine dunkle Socke anziehen, so dass die Konturen bzw. der Umriss seines Fußes vor dem weißen DIN A4-Blatt besonders gut erkennbar sind. Dies vereinfacht die anschließende automatisierte Bildverarbeitung.

Es ist bevorzugt, dass nach dem Fotografieren des Fußes und vor dem Errechnen seiner Abmessungen die folgenden Verfahrensschritte durchgeführt werden:
Zunächst wird eine perspektivische Verzerrung, sofern eine solche vorhanden ist, aus dem aufgenommenen Foto herausgerechnet. Eine perspektivische Verzerrung hat beispielsweise zur Folge, dass die Kanten eines ursprünglich rechteckigen Gegenstandes, beispielsweise eines DIN A4-Blattes, auf dem Foto nicht parallel zueinander verlaufen, sondern sich in einem Fluchtpunkt treffen. Um die genauen Abmessungen eines Fußes zuverlässig bestimmen zu können, kann eine solche perspektivische Verzerrung durch bekannte Bildverarbeitungsalgorithmen aus dem aufgenommenen Bild herausgerechnet werden.

Entsprechendes gilt für eine Verzeichnung, die durch die verwendete Optik der Kamera verursacht wird. Je nach verwendeter Optik und Abstand zum aufgenommenen Objekt kann eine tonnen- oder kissenförmige Verzeichnung vorliegen, die ebenfalls mittels bekannter Bildverarbeitungsalgorithmen aus dem aufgenommenen Foto herausgerechnet werden kann.

Nach diesem Herausrechnen der perspektivischen Verzerrung und/ oder der Verzeichnung wird ein Abbild generiert, das diese Verzerrung oder Verzeichnung nicht mehr aufweist. In weiteren Schritten können dann anhand dieses Abbildes die genauen Abmessungen des Fußes bzw. des aufgezeichneten Umrisses berechnet werden.

Weiter im Verlauf des erfindungsgemäßen Verfahrens können die Konturen des Fußes oder des aufgezeichneten Umrisses des Fußes unter Verwendung eines Konturenfindungsalgorithmus, wie er beispielsweise aus dem Stand der Technik bekannt ist, aufgefunden werden. Z.B. kann der Canny-Algorithmus verwendet werden. Zum Auffinden der Konturen ist es vorteilhaft, wenn der Fuß vor einem Hintergrund aufgenommen wird, der einen hohen Kontrast gegenüber dem Fuß aufweist bzw. ein Foto zur Verfügung steht, das den Aufnahmeort ohne die zu vermessende Person darstellt.

Weiterhin werden Störkonturen, die auf dem aufgenommenen Foto sichtbar sind, herausgerechnet. Hierbei kann es sich beispielsweise um Knicke im Papier handeln. Weiterhin können neben dem aufgenommenen Fuß weitere Konturen auf dem aufgenommenen Foto sichtbar sein, die jedoch nicht den Umriss des Fußes darstellen und somit für das erfindungsgemäße Verfahren nicht weiter berücksichtigt werden müssen. Derartige Konturen sollen somit eliminiert werden. Ein mögliches Kriterium hierzu ist, dass sämtliche Störkonturen, die eine Länge von weniger als 5 mm aufweisen, herausgerechnet werden.

In einer bevorzugten Ausführungsform werden beim Auffinden des aufgezeichneten Umrisses des Fußes folgende Verfahrensschritte im Rahmen eines Edge-Detection-Algorithmus verwendet: Zunächst wird das aufgenommene Foto in ein Graustufenbild umgewandelt. Anschließend wird ein Tonwert-Histogramm des aufgenommenen Fotos erstellt. In der digitalen Bildverarbeitung versteht man unter einem Histogramm die statistische Häufigkeit der Grauwerte bzw. der Farbwerte in einem Bild. Anschließend wird ein Schwarz-Weiß-Bild aus dem aufgenommenen Foto erstellt, wobei jeder Bildpunkt im aufgenommenen Foto, der eine Helligkeit aufweist, die niedriger als ein vorgewählter Helligkeitsschwellwert ist, als schwarzer Bildpunkt definiert wird und die restlichen Bildpunkte als weiße Bildpunkte definiert werden. Das hiermit erstellte Schwarz-Weiß-Bild weist somit lediglich vollständig schwarze oder vollständig weiße Bildpunkte und bevorzugt keine Graustufen auf. Als Helligkeitsschwellwert kann beispielsweise der Median des Tonwert-Histogramms vorgewählt werden. Z.B. kann die Verteilung der Grauwerte im Bild zur Kalkulation des unteren bzw. oberen Schwellwerts für den Canny-Algorithmus genutzt werden. Durch die individuelle Berechnung der Schwellenwerte für jedes Foto kann eine bessere Segmentierung des Bildes erreicht werden als bei der Nutzung von vorgegebenen Standardwerten. Es kann somit eine höhere Trefferquote bei der Kantenfindung erzielt werden.

Es ist bevorzugt, dass das Referenzobjekt flächig ausgebildet ist, d.h. dass es in Bezug auf seine Höhe eine sehr große Breite und Länge aufweist. Weiterhin kann das Referenzobjekt rechteckförmig ausgebildet sein. Wie bereits dargestellt, eignet sich als Referenzobjekt insbesondere ein weißes DIN A4-Blatt bzw. jedes normierte Format, das den vollständigen Umriss aufnehmen kann. Eine auf dem Foto nicht sichtbare Ecke des rechteckförmigen Referenzobjekts kann durch den Schnittpunkt der Verlängerungen der benachbarten Kanten des Referenzobjekts berechnet werden, sofern diese Ecke auf dem Bild durch ein Körperteil des Nutzers oder einen anderen Gegenstand verdeckt ist. Beispielsweise kann bei der Verwendung eines DIN A4-Blattes als Referenzobjekt je nach Aufnahmewinkel eine der Ecken des DIN A4-Blattes durch den Unterschenkel des Nutzers verdeckt sein. Zur weiteren Bildverarbeitung und insbesondere zur Bestimmung des Abstandes und des Winkels des Referenzobjekts zur Kamera ist es vorteilhaft, alle vier Ecken des rechteckförmigen Referenzobjekts zu kennen.

Bei einem direkten Fotografieren des Fußes des Nutzers kann es passieren, dass der Unterschenkel des Nutzers ebenfalls auf der Fotografie sichtbar ist. Der Unterschenkel kann jedoch hinderlich für die genaue Berechnung der Abmessungen des Fußes sein, so dass es notwendig ist, den aufgenommenen Unterschenkel aus der Fotografie herauszurechnen. Dies kann durch folgende Schritte geschehen: Zunächst wird der gemeinsame Umriss des Fußes und des Unterschenkels durch einen Edge-Detection-Algorithmus bestimmt. Anschließend werden die zwei Übergangspunkte, an denen der Umriss des Fußes in den Umriss des Unterschenkels übergeht, an den zwei Stellen, an denen der gemeinsame Umriss des Fußes und des Unterschenkels eine Beugung aufweist, die einen vorbestimmten Knick-Schwellwert überschreitet, bestimmt. Diese beiden Übergangspunkte werden zum Erzeugen eines in sich geschlossenen Umrisses des Fußes miteinander verbunden.

Bei einem Fotografieren des aufgezeichneten Umrisses des Fußes auf einer Darstellungsfläche kann es passieren, dass der aufgezeichnete Umriss keinen vollständig geschlossenen Kreis oder Umriss bildet. Anders ausgedrückt, sind der Startpunkt und der Endpunkt des Umrisses nicht identisch. Unter dem Startpunkt wird derjenige Punkt verstanden, an dem der Nutzer mit einem Stift angesetzt hat, um den Umriss aufzuzeichnen. Der Endpunkt ist entsprechend der Punkt, an dem der Nutzer den Stift wieder von der Darstellungsfläche entfernt hat. In einem solchen Fall kann der Umriss des Fußes durch einen Edge-Detection-Algorithmus durch folgende Schritte erkannt werden: Zunächst werden die Positionen des Startpunktes und des Endpunktes des Umrisses durch den Konturenfindungs-Algorithmus bestimmt. Der Konturenfindungs-Algorithmus liefert, vereinfacht ausgedrückt, eine geordnete Punktekette, aus welcher der erste und letzte Punkt einfach extrahiert werden können. Gleiches gilt auch für den zweiten und den vorletzten Punkt usw. Somit lassen sich Verbindungsvektoren zwischen diesen Punkten bestimmen (Start → Start + 1; Ende → Ende - 1). Es wird davon ausgegangen, dass die Verbindungsvektoren gegeneinander zeigen, wenn sie den Anfang und das Ende einer Kontur darstellen.

Es wird bei einem bestimmten Intervall der Betrag der durchschnittlichen Richtungsänderungen der Vektoren bestimmt und mit der Richtung des Verbindungsvektors zwischen dem Endpunkt und dem Startpunkt verglichen, um sicherzustellen, dass die bestimmte Verbindung sinnvoll ist. Anschließend wird der Abstand der Punkte untersucht, wobei ein Schwellenwert berücksichtigt werden kann. Dieser kann in Abhängigkeit von der Größe des verwendeten Referenzobjekts, der Bildgröße und weiterer hieraus resultierender Relationen bestimmt werden.

Der Algorithmus für die Vermessung eines Fußes kann beispielsweise folgendermaßen ablaufen:
Zunächst erfolgt eine Korrektur von Verzerrung und Verzeichnung durch die Nutzung des Referenzobjekts. Dieses dient zur Ermittlung eines entzerrten Koordinatensystems. Das Referenzobjekt wird anschließend als neue Region of Interest (ROI) extrahiert. Dies bedeutet, dass von nun an nur noch mit diesem Bildabschnitt gearbeitet wird. Diese ROI wird nun in ein Graustufenbild umgewandelt. Weitere Merkmale bezüglich dieses Verfahrensschrittes werden in den folgenden Absätzen beschrieben. Es kann hierbei das Histogramm der Helligkeitswerte des aufgenommenen Bildes ermittelt werden. Anschließend erfolgt eine Kalkulation des oberen und unteren Schwellenwertes zur verbesserten Edge-Detection, z.B. durch den Canny-Algorithmus, welcher diese Werte benötigt. Anschließend erfolgt die eigentliche Edge-Detection durch den Canny-Algorithmus mit den dynamisch ermittelten Schwellwerten. Das daraus resultierende Binärbild wird dann für die Konturenfindung genutzt. Die Konturenfindung kann beispielsweise durch openCV-Bordmittel (z.B. Suzuki85-Algorithmus) erfolgen. Nun kann eine Längenfilterung der Konturen erfolgen. Der Schwellenwert ist abhängig von der Größe des Referenzobjekts, der Bildgröße und weiterer hieraus resultierender Relationen. Durch diesen Schritt werden Störlinien, die für die Größenbestimmung des Umrisses des Fußes hinderlich sind, beseitigt. Anschließend wird die Kontur durch Angleichung der Polygonkurven vereinheitlicht. Sofern der Start- und der Endpunkt der Kontur nicht identisch sind, kann der Umriss geflickt werde, indem der vorliegend beschriebene Algorithmus verwendet wird.

Bevorzugt kann das erfindungsgemäße Verfahren auch Schritte zum Bestimmen der Größe und/ oder der Abmessungen eines Kleidungsstücks umfassen.

Kleidungsstücke können aufgrund von Fertigungstoleranzen oder aufgrund ihrer Machart variierende Abmessungen aufweisen und von den standardisierten konfektionsgrößenspezifischen Abmessungen abweichen, auch wenn sie mit einer bestimmten Konfektions- oder Schuhgröße ausgezeichnet sind. Um einem Nutzer, der ein solches Kleidungsstück beispielsweise im Versandhandel bestellen möchte und dieses somit nicht unmittelbar anprobieren kann, ein passendes Kleidungsstück vorschlagen zu können, ist es wünschenswert, die genauen Abmessungen eines Kleidungsstücks individuell ermitteln zu können. Individuell kann beispielsweise bedeuten, dass ein Kleidungsstück, das mit einer bestimmten Größe ausgezeichnet ist, dennoch individuell vermessen wird und alle Kleidungsstücke dieser Machart mit den dann ermittelten Abmessungen versehen werden. Zusätzlich ist es möglich, sämtliche Kleidungsstücke derselben Machart, d.h. wirklich jedes einzelne Kleidungsstück, zu vermessen, um auch Fertigungstoleranzen zu berücksichtigen.

Zunächst wird eine Abbildung des Kleidungsstücks und eines Referenzobjekts mit bekannten Abmessungen erstellt. Hierbei sind das Kleidungsstück und das Referenzobjekt auf derselben Abbildung abgebildet. Ferner sind das Kleidungsstück und das Referenzobjekt während des Erstellens der Abbildung in einer gemeinsamen Ebene oder in zwei Ebenen, deren Abstand und Winkel zueinander bekannt sind, angeordnet.

Anschließend werden die Abmessungen und/ oder die Größe des Kleidungsstücks anhand der aufgenommenen Abbildungen basierend auf den bekannten Abmessungen des Referenzobjekts errechnet. Unter der errechneten Größe des Kleidungsstücks wird in diesem Zusammenhang verstanden, dass z.B. die angegebene Konfektions- oder Schuhgröße eines Kleidungsstücks korrigiert werden kann. Wird beispielsweise ein Schuh, der ursprünglich mit der Schuhgröße 42 ausgezeichnet war, durch das erfindungsgemäße Verfahren vermessen und ergibt die Messung, dass er nur die Abmessungen eines Schuhs der Größe 41 aufweist, so kann die angegebene Größe entsprechend korrigiert werden.

Eine noch genauere Zuordnung ist allerdings möglich, wenn nicht lediglich die Größe korrigiert wird, sondern wenn mit konkreten Abmessungen, d.h. in diesem Fall mit der Länge und Breite des Kleidungsstücks, die beispielsweise in Millimeter angegeben werden können, gearbeitet wird. Auch ist es möglich, nicht nur die maximale Länge und Breite anzugeben, sondern den genauen Verlauf des Umrisses des Kleidungsstücks, so dass die Abmessungen des Kleidungsstücks an jeder beliebigen Stelle bekannt sind.

Hieraus resultiert eine Bestimmung der richtigen/passenden Konfektionsgröße anhand einer Korrelation zwischen Körperabmessungen und Textilabmessungen und eine anschließende Größenempfehlung kann verlässlich erfolgen.

Das Erstellen der Abbildung des Kleidungsstücks kann durch verschiedene Technologien vorgenommen werden. Im einfachsten Fall kann eine Foto- oder Videokamera verwendet werden. Für bestimmte Kleidungsstücke, wie beispielsweise Schuhe, unterscheiden sich die Außenabmessungen von den Innenabmessungen, wobei je nach Machart des Schuhs möglicherweise von den gemessenen Außenabmessungen nicht auf die relevanten Innenabmessungen geschlossen werden kann. Zur Erfassung der Innenabmessungen können andere Technologien, wie beispielsweise die Computertomographie oder Röntgenstrahllung, verwendet werden. Weitere alternative Abbildungsverfahren sind die Lichtschnitttechnologie, Laserprofilometrie, Streifenprojektion, die Thermographie oder die Verwendung von Mikrowellen.

Zum Bestimmen der genauen Innenabmessungen, insbesondere der Innenlänge und der Innenbreite eines Schuhs, kann neben dem Erstellen einer Abbildung des Schuhs auch eine Abbildung der herausgenommenen Innensohle des Schuhs erstellt werden. Dies kann ebenfalls im Rahmen des oben beschriebenen Verfahrens geschehen, so dass die Innensohle des Schuhs gleichzeitig mit dem Referenzobjekt abgebildet wird. Es kann mit den obengenannten Technologien sowohl ein 2- als auch ein 3-dimensionales Abbild des Schuhinnenraumes erfolgen, welches für die Größenbestimmung herangezogen wird.

Beispielsweise ist es möglich, bei zwei definierten Schuhgrößen, beispielsweise bei der größten und kleinsten Schuhgröße, sowohl den Schuh als auch die Innensohle abzubilden und zu vermessen, ebenso kann eine Korrelation zwischen Außenabmessungen und dem 2- bzw. 3-dimensionalen Abbild erstellt werden. Anhand dieser Abbildungen kann ermittelt werden, wie weit die Innenschuhabmessungen, die anhand der abgebildeten Einlegesohle bestimmt werden können, von den Außenabmessungen des Schuhs abweichen. Diese ermittelte Abweichung kann für die dazwischenliegenden Schuhgrößen verwendet werden, so dass hier lediglich der Schuh und nicht die Innensohle abgebildet werden muss.

In einer bevorzugten Ausführungsform wird neben einer Darstellung des Kleidungsstücks aus einer direkten Perspektive mindestens eine weitere Darstellung in einer weiteren Perspektive unter Verwendung mindestens eines Reflektorelements, insbesondere eines Spiegels, abgebildet. Somit können die Größe und/oder die Abmessungen des Kleidungsstücks in mindestens zwei oder sogar drei Ebenen erfasst werden.

Beispielsweise ist es hierdurch möglich, einen Schuh auch von vorne oder von der Seite aufzunehmen, um so seinen Höhenverlauf festzustellen. Es kann somit herausgefunden werden, ob dieser Schuh beispielsweise für Menschen mit einem hohen oder flachen Spann geeignet ist.

Besonders vorteilhaft ist es, wenn die vorliegend beschriebenen Verfahren zum Bestimmen der Passgenauigkeit eines Kleidungsstücks miteinander kombiniert werden. Hierzu werden die Abmessungen des Körperteils eines Nutzers gemäß dem ersten beschriebenen Verfahren bestimmt. Die Größe und/oder die Abmessungen mindestens eines Kleidungsstücks können automatisiert bestimmt werden. Hierbei ist es bevorzugt, dass die Größe und/oder die Abmessungen einer größeren Zahl von Kleidungsstücken bestimmt werden.

Im Folgenden wird mindestens ein passendes Kleidungsstück, dessen gemessene Größe und/oder Abmessungen mit den gemessenen Abmessungen des Fußes übereinstimmen, ausgegeben. Je mehr Kleidungsstücke vermessen wurden, desto größer ist die Wahrscheinlichkeit, dass ein oder mehrere Kleidungsstücke existieren, die als passend ausgegeben werden können.

Das Ausgeben kann beispielsweise durch Anzeigen eines Bildes und/oder einer Beschreibung des Kleidungsstücks auf einer Anzeigevorrichtung erfolgen. Bei dieser Anzeigevorrichtung kann es sich beispielsweise um einen Computer, ein Smartphone, einen Tablet-PC oder ein ähnliches elektronisches Gerät handeln. Auch kann das Ausgeben eines passenden Kleidungsstücks in einem Teleshoppingkanal oder im stationären Handel erfolgen.

In besonders vorteilhafter Weise findet der Teil des Verfahrens, bei dem die Abmessungen des Fußes eines Nutzers bestimmt werden, lokal in einer Software statt, die auf einem Endgerät des Nutzers, z.B. einem Smartphone, einem Computer, etc. installiert ist. Hierfür muss dieser Software lediglich ein Foto des Fußes oder des aufgezeichneten Umrisses des Fußes zur Verfügung gestellt werden. Dieses Foto kann durch eine Digitalkamera aufgenommen werden und der Software anschließend zur Verfügung gestellt werden. Besonders vorteilhaft ist es, hierzu die Digitalkamera eines Smartphones zu verwenden, so dass das aufgenommene Bild direkt intern an die auf dem Smartphone installierte Software übergeben bzw. an einen Server gesendet und ausgewertet werden kann.

Es ist ferner bevorzugt, dass das verwendete Endgerät mit dem Internet verbindbar ist und die bestimmten Abmessungen des Fußes an ein Hintergrundsystem übermitteln kann. Bei diesem Hintergrundsystem kann es sich beispielsweise um den Server eines Onlineversenders von Kleidungsstücken handeln. Auf diesem Server oder auf einem mit diesem verbundenen Rechner kann eine Software installiert sein, die mindestens ein passendes Kleidungsstück ermittelt, und Informationen hierüber, beispielsweise ein Bild und/oder eine Beschreibung, über das Internet oder ein anderes Datennetzwerk an das Endgerät des Nutzers zurückschickt. Der Nutzer kann sodann direkt auf seinem Endgerät eines der ermittelten Kleidungsstücke auswählen und bestellen.

Die Software des Hintergrundsystems ist mit einer Datenbank verbindbar, auf der die gemessene Größe und/oder die gemessenen Abmessungen einer Vielzahl von Kleidungsstücken hinterlegt sind, so dass ein Vergleich mit den gemessenen Abmessungen des Fußes des Nutzers erfolgen kann.

Das beschriebene Verfahren zum Bestimmen der Größe und/oder der Abmessungen eines Kleidungsstücks kann neben dem zuletzt beschriebenen Verfahren auch im Rahmen eines Qualitätssicherungsprozesses verwendet werden. Hierzu können zum Zwecke der Qualitätssicherung die Größe und/ oder die Abmessungen eines Kleidungsstücks automatisiert, beispielsweise direkt nach der Produktion ermittelt werden. Bisher erfolgte dies lediglich stichprobenartig und wird manuell durchgeführt, so dass ein relativ großer Aufwand erzeugt wird. Durch das beschriebene Verfahren ist es möglich, alle produzierten Kleidungsstücke zu vermessen und die Kleidungsstücke erst nach durchgeführter Vermessung mit einem Etikett für die Größe zu versehen.

Neben der Größe und/oder den Abmessungen der Kleidungsstücke können durch das beschriebene Verfahren auch weitere Eigenschaften von Kleidungsstücken automatisiert ermittelt werden. Beispielsweise kann automatisiert erfasst werden, um welches Kleidungsstück es sich handelt. Dies kann im Rahmen der Verarbeitung von Retouren von großer Bedeutung sein. Onlineversender sehen sich mit einer äußerst großen Anzahl von Retouren konfrontiert, die kontrolliert werden müssen. Dies erfolgt bisher je nach Wert des zurückgeschickten Artikels unter Umständen lediglich stichprobenartig. Durch das erfindungsgemäße Verfahren ist es möglich, sämtliche zurückgeschickten Artikel automatisch zu erfassen und herauszufinden, ob es sich bei diesem Artikel tatsächlich um den angegebenen Artikel handelt. Somit kann Betrugsversuchen vorgebeugt werden, bei denen ein Käufer einen günstigeren Artikel zurückschickt, sich aber das Geld für einen teureren Artikel erstatten lässt bzw. dieser nicht in Rechnung gestellt wird.

Es wird ferner eine Vorrichtung zum Bestimmen der Größe und/oder der Abmessungen eines Kleidungsstücks offenbart, die im Rahmen der beschriebenen Verfahren angewendet werden kann, die aber als solche nicht Gegenstand der durch die Ansprüche definierten Erfindung ist. Die Vorrichtung weist einen Aufnahmeraum zum Aufnehmen des Kleidungsstücks auf. Hierbei kann es sich im einfachsten Fall um eine Aufnahmefläche handeln, auf die das Kleidungsstück aufgelegt wird. Unter einem Aufnehmen des Kleidungsstücks wird ein räumliches Aufnehmen verstanden. Der Aufnahmeraum ist somit ein Raum, in den das Kleidungsstück aufgenommen werden kann.

Die Vorrichtung umfasst ferner eine Abbildungsvorrichtung zum Aufnehmen einer Abbildung des Kleidungsstücks. Die Abbildungsvorrichtung erstellt somit eine Abbildung des Kleidungsstücks, die anschließend zum Zwecke des Ermittelns der Abmessungen des Kleidungsstücks weiterverarbeitet wird. Die Abbildungsvorrichtung ist in einem definierten Abstand und Winkel zum Aufnahmeraum fixierbar. Die Abbildungsvorrichtung kann in einem festen Abstand und Winkel zum Aufnahmeraum angeordnet oder auch verstellbar sein. Der Abstand und Winkel zum Aufnahmeraum sind wichtig, um perspektivische Verzerrungen und die Verzeichnung des aufgenommenen Kleidungsstücks zu minimieren oder zu vermeiden.

Die Vorrichtung umfasst ferner ein Referenzobjekt mit bekannten Abmessungen, das derart angeordnet ist, dass es durch die Abbildungsvorrichtung gleichzeitig mit dem Kleidungsstück erfassbar ist. Das Referenzobjekt kann beispielsweise auf dem Aufnahmeraum oder in der Nähe dessen angeordnet sein.

In einer bevorzugten Ausführungsform weist die Vorrichtung mindestens ein Reflektorelement auf, durch das Strahlung, die vom Kleidungsstück nicht in Richtung der Abbildungsvorrichtung reflektiert oder ausgestrahlt wurde, in Richtung der Abbildungsvorrichtung definiert umgelenkt wird. Somit ist neben einer Darstellung des Kleidungsstücks aus einer direkten Perspektive mindestens eine weitere Darstellung in einer weiteren Perspektive möglich. Bei dem mindestens einen Reflektorelement kann es sich insbesondere um einen Spiegel handeln. Dieser kann beispielsweise relativ zur Aufnahmefläche in einem Winkel von 45° angeordnet sein, so dass er eine Front- oder Seitenansicht des Kleidungsstücks in Richtung der Abbildungsvorrichtung reflektiert.

Als Alternative zu den bisher beschriebenen Verfahren ist es auch möglich, dass ein Nutzer unter Verwendung einer Digitalkamera und einer hiermit verbindbaren Software ein Kleidungsstück fotografiert, dessen Größe und/oder Abmessungen automatisch bestimmt werden. Hierbei kann es sich beispielsweise um ein Kleidungsstück handeln, dass dem Nutzer besonders gut passt. Die gemessenen Abmessungen werden sodann über ein Datennetz an ein Hintergrundsystem übermittelt, wobei das Hintergrundsystem ein oder mehrere Kleidungsstücke, die ebenfalls die gemessenen Abmessungen aufweisen, als Abbildung und/oder Beschreibung an den Nutzer übermittelt. Auf diese Weise ist es dem Nutzer möglich, ein Kleidungsstück mit Abmessungen zu bestellen, die ähnlich zu den Abmessungen eines Kleidungsstücks mit einer guten Passform sind, das er bereits besitzt.

Es ist ferner möglich, dass die Software, die auf dem Endgerät des Nutzers installiert ist, eine erstellte Fotografie des Fußes oder des Umrisses an das Hintergrundsystem übermittelt, sofern die Abmessungen des Fußes und/oder des Umrisses nicht zuverlässig ermittelt werden können. Dies kann beispielsweise dann vorkommen, wenn der Edge-Detection-Algorithmus nicht in der Lage ist, den Umriss des Fußes zu erkennen. Diese an das Hintergrundsystem übermittelten Fotos können ausgewertet werden, wodurch im Rahmen eines Lernprozesses die Software und insbesondere der Edge-Detection-Algorithmus verbessert werden können.

Beim Erstellen eines Fotos eines Fußes des Nutzers ist es auch möglich, eine zusätzliche Aufnahme aus einem etwas anderen Winkel zu erstellen. Beispielsweise kann die erste Aufnahme in einem Winkel von der rechten Seite erfolgen, während die zweite Aufnahme den Fuß in einem Winkel von der linken Seite aufnimmt. Hierdurch kann die Störung, die durch den abgebildeten Unterschenkel des Nutzers entsteht, minimiert werden.

Weiterhin ist es möglich, die Ferse des Fußes an eine Wand zu platzieren und ein einziges Bild von oben zu machen. Auf diesem Bild ist auch der untere Rand der Wand sichtbar, so dass die Länge des Fußes auf sehr einfache Weise ermittelt werden kann.

Im Folgenden werden bevorzugte Ausführungsformen anhand von Figuren erläutert.

Es zeigen:
- Fig. 1: eine schematische Ansicht eines aufgenommenen Körperteils,
- Fig. 2 u. 3: schematische Ansichten des Umrisses eines aufgezeichneten Körperteils,
- Fig. 4: eine beispielhafte Ausführungsform eines Aufnahmeraums zum Aufnehmen eines Kleidungsstücks,
- Fig. 5: eine beispielhafte Ausführungsform der erfindungsgemäßen Vorrichtung,
- Fig. 6: verschiedene Körperteile, deren Abmessungen ermittelt werden können (nicht erfindungsgemäß),
- Fig. 7: ein Kleidungsstück, das vor einer Vielzahl von Referenzobjekten fotografiert wurde (nicht erfindungsgemäß), und
- Fig. 8: eine Fotografie, die im Rahmen eines nicht erfindungsgemäßen Verfahrens aufgenommen wurde.

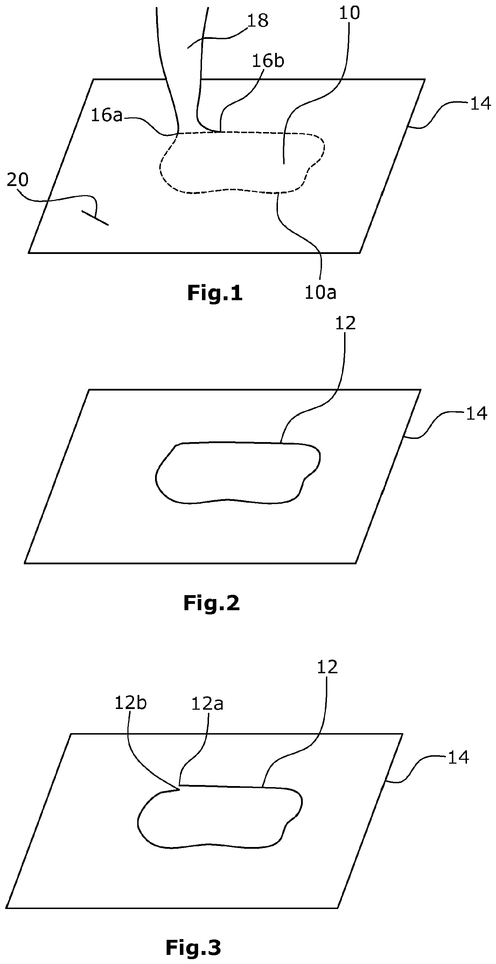

In Fig. 1 ist als Referenzobjekt 14 ein DIN A4-Blatt dargestellt. Auf diesem DIN A4-Blatt hat der Nutzer seinen Fuß 10 platziert. Fig. 1 stellt schematisch ein Foto dar, das der Nutzer von seinem Fuß 10 und dem DIN A4-Blatt 14 erstellt hat. Auf dem Foto ist auch der Unterschenkel 18 des Nutzers sichtbar. Auf dem Foto ist ferner auf dem DIN A4-Blatt 14 eine Störkontur 20, die beispielsweise durch einen Knick im Blatt oder einen nicht zum Umriss des Fußes 10 gehörenden Strich verursacht wird.

In Fig. 1 ist der Umriss 10a des Fußes 10 gestrichelt dargestellt. Dieser wird durch die beschriebenen Verfahrensschritte automatisiert im Rahmen eines Edge-Detection-Algorithmus erfasst.

Zum Bestimmen des vollständigen Umrisses 10a des Fußes 10 ist es wünschenswert, den Unterschenkel 18, der auf dem Bild gemäß Fig. 1 dargestellt ist, herauszurechnen. Hierzu wird der gemessene Umriss des Fußes 10 und des Unterschenkels 18 durch den Edge-Detection-Algorithmus bestimmt. Anschließend werden zwei Übergangspunkte 16a, 16b, in denen der Umriss des Fußes 10 in den Umriss des Unterschenkels 18 übergeht, bestimmt. Diese befinden sich an den zwei Stellen, an denen der gemeinsame Umriss des Fußes 10 und des Unterschenkels 18 eine Beugung aufweist, die einen bestimmten Knick-Schwellwert überschreitet. Anschließend werden die beiden Übergangspunkte 16a, 16b zum Erzeugen eines in sich geschlossenen Umrisses 10a des Fußes 10 miteinander verbunden.

In Fig. 2 ist der Umriss 12 eines Fußes auf ein DIN A4-Blatt 14 aufgezeichnet. Das DIN A4-Blatt dient hierbei gleichzeitig als Referenzobjekt und Darstellungsfläche. In Fig. 2 ist ein mögliches Foto dargestellt, dass ein Nutzer von dem DIN A4-Blatt 14 gemacht hat, wobei hierauf der Fuß 10 des Nutzers an sich nicht dargestellt ist. Unter Verwendung der beschriebenen Verfahrensschritte können anhand des aufgezeichneten Umrisses 12 die Abmessungen des Fußes 10 des Nutzers bestimmt werden.

Dies ist auch dann möglich, wenn gemäß Fig. 3 der Umriss 12 nicht vollständig geschlossen ist, d.h. wenn der Startpunkt 12a und der Endpunkt 12b des Umrisses nicht identisch sind.

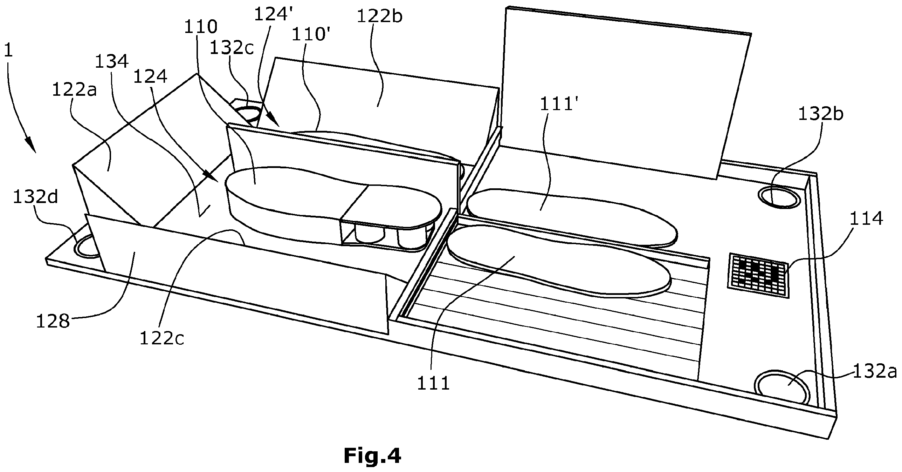

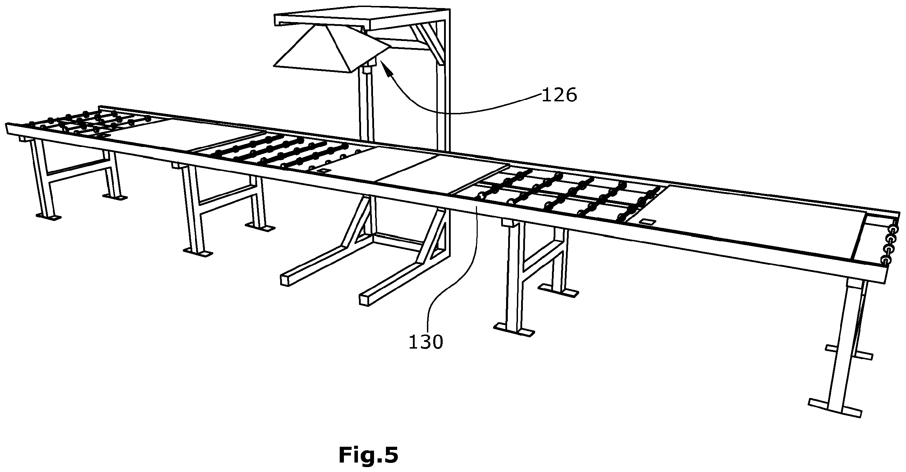

In Fig. 4 ist ein beispielhafter Aufnahmeraum 124, 124' dargestellt, in den z.B. Schuhe 110, 110' aufgenommen werden können. Über diesem Aufnahmeraum 124, 124' ist eine in Fig. 4 nicht dargestellte Abbildungsvorrichtung 126, beispielsweise eine Fotokamera, angeordnet. Eine entsprechende Abbildungsvorrichtung 126 ist in Fig. 5 dargestellt.

Der Aufnahmeraum 124, 124' kann in einem Gehäuse 128 ausgebildet sein. Dieses Gehäuse 128 kann zum Erstellen der Abbildung auf einem Laufband 130 platziert werden, das in Fig. 5 dargestellt ist. Das Gehäuse 128 und somit auch der Aufnahmeraum 124, 124' können somit in einem definierten Abstand und Winkel zu der Abbildungsvorrichtung 126 angeordnet werden. Sobald sich der Aufnahmeraum 124, 124' unter der Abbildungsvorrichtung 126 befindet, wird eine Abbildung des Aufnahmeraums 124, 124' erstellt. Hierbei kann auch der hintere Teil des Gehäuses 128 abgebildet werden, in dem die beiden Einlegesohlen 111, 111' angeordnet sind, die zu den Schuhen 110, 110' gehören, die im Aufnahmeraum 124, 124' angeordnet sind. An einer beliebigen Stelle des Aufnahmeraums 124, 124' bzw. des Gehäuses 128 ist ein Referenzobjekt 114 mit bekannten Abmessungen angeordnet. Weiterhin kann das Gehäuse 128 Markierungen 132a - 132d aufweisen, durch die die Ausrichtung des Gehäuses 128 und somit auch des Aufnahmeraums 124, 124' und der Kleidungsstücke 110, 110' automatisiert erfasst werden kann.

Um den Aufnahmeraum 124, 124' herum sind drei Spiegel 122a - 122c angeordnet, die vorzugsweise einen Winkel von 45° relativ zur Grundfläche 134 des Aufnahmeraums 124, 124' aufweisen. Durch die Spiegel 122b und 122c wird die Seitenansicht der Kleidungsstücke 110, 110' in Richtung der Kamera 126 reflektiert, während durch den Spiegel 122a die Frontansicht reflektiert wird.

Durch die in Fig. 5 dargestellte Vorrichtung ist ein automatisiertes Erfassen der Größen und/oder Abmessungen einer Vielzahl von Kleidungsstücken möglich. Hierzu ist ein Laufband 130 vorgesehen, auf dem die Kleidungsstücke beispielsweise unter Verwendung eines Gehäuses 128, das einen Aufnahmeraum beherbergt, an der Kamera 126 vorbeigeführt werden können.

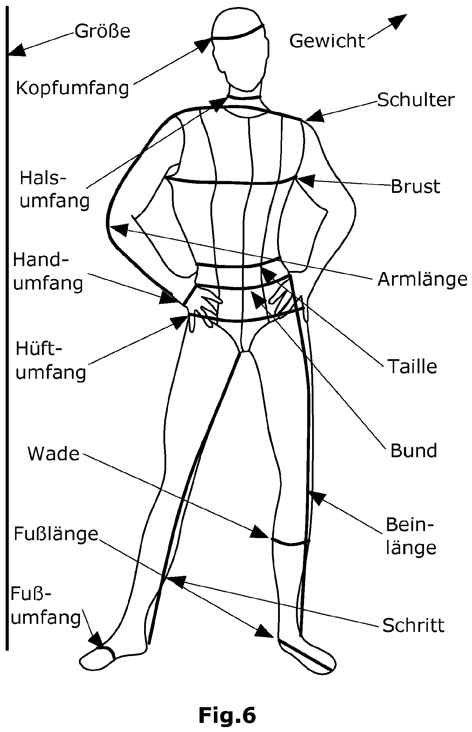

Fig. 6 zeigt den Körper eines Nutzers, wobei durch das Verfahren eine, mehrere oder alle genannten Abmessungen der Körperteile ermittelt werden können. Als Referenzobjekt 14 kann hierbei die bekannte Größe des Nutzers verwendet werden. Es ist somit auf einfache Weise möglich, die Abmessungen einer Vielzahl von Körperteilen gleichzeitig zu ermitteln, wobei hier vorzugsweise nur ein einziges Referenzobjekt verwendet werden muss.

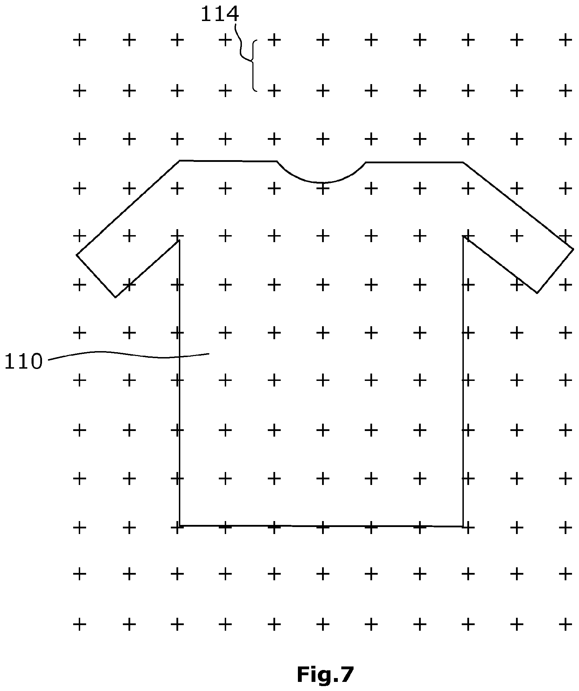

Fig. 7 zeigt ein Kleidungsstück 110, in diesem Fall ein T-Shirt, das vor einer Vielzahl von Referenzobjekten 114 fotografiert wurde. Bei dem in Fig. 7 dargestellten Referenzobjekt 114 handelt es sich um den Abstand zwischen zwei benachbarten Kreuzen.

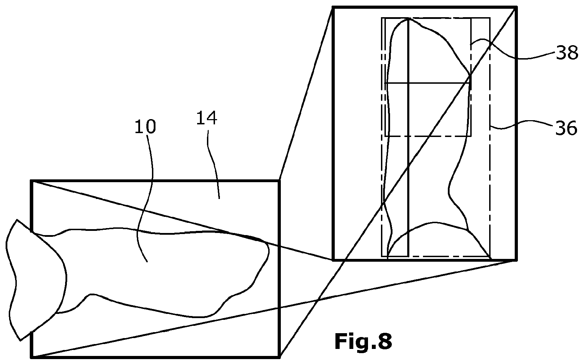

Fig. 8 stellt eine Fotografie dar, die im Rahmen eines nicht erfindungsgemäßen Verfahrens aufgenommen wurde. Bei diesem nicht erfindungsgemäßen Verfahren kann das Körperteil vor dem Referenzobjekt fotografiert werden, wobei das hintere Ende des Körperteils genau mit der hinteren Kante des Referenzobjekts 14 abschließt. Sofern es sich beispielsweise bei dem Referenzobjekt um ein Blatt Papier handelt und das Körperteil der Fuß 10 eines Nutzers ist, kann der Fuß 10 mit der Ferse genau an der hinteren Kante des Blattes positioniert und von oben herab fotografiert werden. Nun wird die bekannte Findung des Referenzobjekts angewendet. Die Verzeichnung und Verzerrung kann herausgerechnet werden, wie es bereits in Zusammenhang mit dem bisher beschriebenen Verfahren dargestellt wurde. Auch die anderen Merkmale des bisher beschriebenen Verfahrens können in dieser Ausführungsform Anwendung finden. Es wird nun, beginnend mit dem hinteren Ende des Referenzobjekts, ein Rechteck 36 generiert, das die Länge des zu vermessenden Körperteils kennzeichnet. Hierbei handelt es sich um das kleinste Rechteck, in dem sich das abgebildete Körperteil darstellen lässt. Ein derartiges Rechteck wird auch als Bounding-Box bezeichnet. Die Breite des Körperteils kann nicht durch dieses Rechteck bestimmt werden, da z.B. in Fig. 8 Teile das abgebildete Bein am unteren Bildrand dieses Ergebnis verfälschen würden. Daher wird die Kontur des Körperteils auf der Hälfte ihrer Länge in zwei Teile geteilt. Der obere Teil wird mit einem zweiten Rechteck 38 umzogen, um die tatsächliche Breite des Körperteils zu bestimmen.

## Patentansprüche

1. Verfahren zum Bestimmen der Abmessungen mindestens eines Fußes eines Nutzers, mit den folgenden Verfahrensschritten:
a) Platzieren wenigstens eines Fußes eines Nutzers auf einem Referenzobjekt mit bekannten Abmessungen, wobei als Referenzobjekt ein weißes Blatt mit einem normierten Format verwendet wird,
b) Fotografieren des Fußes oder des auf dem Referenzobjekt aufgezeichneten Umrisses (12) des Fußes und des Referenzobjekts mit einer Digitalkamera eines Mobilfunkendgeräts, beispielsweise eines Smartphones, wobei der Fuß oder der Umriss (12) und das Referenzobjekt auf demselben Foto abgebildet sind,
c) Errechnen der Abmessungen des Fußes anhand der Fotografie basierend auf den bekannten Abmessungen des Referenzobjekts vermittels einer Software, die als Applikation auf dem Mobilfunkendgerät oder einem Server installiert ist, wobei Störkonturen, die auf dem aufgenommenen Foto sichtbar sind, herausgerechnet werden, wobei das Referenzobjekt und der Fuß zum Zeitpunkt des Fotografierens in einer gemeinsamen Ebene oder in zwei Ebenen, deren Abstand und Winkel zueinander bekannt sind, angeordnet sind.

2. Verfahren nach Anspruch 1, wobei nach dem Fotografieren gemäß Verfahrensschritt b) und vor dem Errechnen der Abmessungen gemäß Verfahrensschritt c) folgende Schritte durchgeführt werden:
- Herausrechnen einer perspektivischen Verzerrung und/oder einer Verzeichnung aus dem aufgenommenen Foto,
- Auffinden der Konturen (10a) des Fußes oder des aufgezeichneten Umrisses (12) des Fußes unter Verwendung eines Konturenfindungsalgorithmus, wobei insbesondere Störkonturen mit einer Länge, die geringer als ein vorbestimmter Schwellwert, insbesondere geringer als 5 mm ist, herausgerechnet werden.

3. Verfahren nach Anspruch 2, wobei beim Auffinden des aufgezeichneten Umrisses (12) des Fußes ein Edge-Detection-Algorithmus mit den folgenden Schritten verwendet wird:
- Erstellen eines Tonwert-Histogramms der Helligkeitsverteilung des aufgenommenen Fotos,
- Erstellen eines Schwarz-Weiß-Bildes aus dem aufgenommenen Foto, wobei jeder Bildpunkt im aufgenommenen Foto, der eine Helligkeit aufweist, die niedriger als ein vorgewählter Helligkeits-Schwellwert ist, als schwarz definiert wird und die restlichen Bildpunkte als weiß definiert werden, wobei insbesondere der Median des Tonwert-Histogramms der Helligkeitsverteilung als Helligkeits-Schwellwert vorgewählt wird.

4. Verfahren nach einem der Ansprüche 1 - 3, wobei das Referenzobjekt flächig ausgebildet ist und eine Rechteckform aufweist, wobei eine auf dem Foto nicht sichtbare Ecke des Rechtecks durch den Schnittpunkt der Verlängerungen der benachbarten Kanten berechnet wird, sofern diese Ecke auf dem Bild durch ein Körperteil des Nutzers oder einen anderen Gegenstand verdeckt ist.

5. Verfahren nach einem der Ansprüche 1 - 4, wobei bei einem direkten Fotografieren des Fußes der Unterschenkel (18) des Nutzers aus der Fotografie durch folgende Schritte herausgerechnet wird, sofern er auf der Fotografie abgebildet ist:
- Bestimmung des gemeinsamen Umrisses des Fußes und des Unterschenkels (18) durch einen Edge-Detection-Algorithmus,
- Bestimmen der zwei Übergangspunkte (16a, 16b), in denen der Umriss des Fußes in den Umriss des Unterschenkels (18) übergeht, an den zwei Stellen, an denen der gemeinsame Umriss des Fußes und des Unterschenkels (18) eine Beugung aufweist, die einen vorbestimmten Knick-Schwellwert überschreitet,
- Verbinden der beiden Übergangspunkte (16a, 16b) zum Erzeugen eines in sich geschlossenen Umrisses (10a) des Fußes.

6. Verfahren nach einem der Ansprüche 1 - 5, wobei bei einem Fotografieren des aufgezeichneten Umrisses (12) des Fußes der Umriss (12) durch einen Kantenfindungs-Algorithmus durch folgende Schritte erkannt wird, auch wenn sein Startpunkt (12a) und sein Endpunkt (12b) nicht identisch sind und er somit keinen vollständig geschlossenen Umriss (12) bildet:
- Bestimmen der Position des Startpunktes (12a) und des Endpunktes (12b) des Umrisses (12) durch den Kantenfindungs-Algorithmus, wobei von einem geschlossenen Umriss (12) ausgegangen wird, wenn der Abstand (12) zwischen dem Startpunkt (12a) und dem Endpunkt (12b) geringer als ein definierter Abstandsschwellwert ist.

7. Verfahren nach einem der Ansprüche 1 bis 6 mit den folgenden zusätzlichen Verfahrensschritten zum Bestimmen der Größe und/ oder der Abmessungen eines Kleidungsstücks (110):
a) Erstellen einer Abbildung des Kleidungsstücks (110) und des Referenzobjekts (114) mit bekannten Abmessungen, wobei das Kleidungsstück (110) und das Referenzobjekt (114) auf derselben Abbildung abgebildet sind und das Kleidungsstück (110) und das Referenzobjekt (114) während des Fotografierens in einer gemeinsamen Ebene oder in zwei Ebenen, deren Abstand und Winkel zueinander bekannt sind, angeordnet sind,
b) Errechnen der Abmessungen und/ oder der Größe des Kleidungsstücks (110) anhand der aufgenommenen Abbildung basierend auf den bekannten Abmessungen des Referenzobjekts (114).

8. Verfahren nach Anspruch 7, wobei auf die Abbildung des Kleidungsstücks (110) neben einer Darstellung des Kleidungsstücks (110) aus einer direkten Perspektive mindestens eine weitere Darstellung aus einer weiteren Perspektive unter Verwendung mindestens eines Reflektorelements (122a, 122b, 122c), insbesondere eines Spiegels, aufgenommen wird, so dass die Größe und/ oder die Abmessungen des Kleidungsstücks (110) in mindestens zwei Ebenen erfasst werden können.

## Claims

1. Method for determining the dimensions of at least one foot of a user, comprising the following method steps:
a) Placing at least one foot of a user on a reference object of known dimensions, the reference object being a white sheet of a standardized format,
(b) photographing the foot or the outline (12) of the foot and the reference object recorded on the reference object with a digital camera of a mobile terminal, for example a smartphone, wherein the foot or the outline (12) and the reference object are imaged on the same photograph,
c) calculating the dimensions of the foot from the photograph based on the known dimensions of the reference object by means of software installed as an application on the mobile terminal or a server, wherein interference contours visible on the captured photograph are subtracted, wherein
the reference object and the foot are located in a common plane or in two planes whose distance and angle to each other are known at the time of photographing.

2. Method according to claim 1, wherein after photographing according to method step b) and before calculating the dimensions according to method step c) the following steps are performed:
- Removal of a perspective distortion and/or a distortion from the captured photo,
- finding the contours (10a) of the foot or the recorded outline (12) of the foot using a contour finding algorithm, wherein in particular interfering contours having a length which is less than a predetermined threshold value, in particular less than 5 mm, are eliminated.

3. Method according to claim 2, wherein in finding the recorded outline (12) of the foot, an edge detection algorithm is used comprising the following steps:
- Create a tonal value histogram of the brightness distribution of the captured photo,
- creating a black and white image from the captured photograph, wherein each pixel in the captured photograph having a brightness lower than a preselected brightness threshold is defined as black and the remaining pixels are defined as white, wherein in particular the median of the tonal value histogram of the brightness distribution is preselected as the brightness threshold.

4. Method according to any one of claims 1 - 3, wherein the reference object is flat and has a rectangular shape, wherein a corner of the rectangle that is not visible on the photograph is calculated by the intersection of the extensions of the adjacent edges, provided that this corner is hidden on the image by a part of the user's body or another object.

5. Method according to any one of claims 1 - 4, wherein when the foot is photographed directly, the user's lower leg (18) is extracted from the photograph by the following steps, if it is depicted in the photograph:
- Determination of the common outline of the foot and the lower leg (18) by an edge detection algorithm,
- determining the two transition points (16a, 16b) at which the outline of the foot merges with the outline of the lower leg (18), at the two points at which the common outline of the foot and the lower leg (18) has a bending which exceeds a predetermined bending threshold,
- Connecting the two transition points (16a, 16b) to create a self-contained outline (10a) of the foot.

6. Method according to any one of claims 1 - 5, wherein upon photographing the recorded outline (12) of the foot, the outline (12) is detected by an edge-finding algorithm by the following steps, even if its starting point (12a) and its ending point (12b) are not identical and thus it does not form a completely closed outline (12):
- determining the position of the start point (12a) and the end point (12b) of the outline (12) by the edge-finding algorithm, wherein a closed outline (12) is assumed if the distance (12) between the start point (12a) and the end point (12b) is less than a defined distance threshold.

7. Method according to any one of claims 1 to 6, comprising the following additional method steps for determining the size and/or dimensions of a garment (110):
a) creating an image of the garment (110) and the reference object (114) with known dimensions, wherein the garment (110) and the reference object (114) are imaged on the same image and the garment (110) and the reference object (114) are arranged in a common plane or in two planes whose distance and angle to each other are known during the photographing,
b) calculating the dimensions and/or size of the garment (110) from the captured image based on the known dimensions of the reference object (114).

8. Method according to claim 7, wherein, in addition to a representation of the garment (110) from a direct perspective, at least one further representation from a further perspective is recorded on the image of the garment (110) using at least one reflector element (122a, 122b, 122c), in particular a mirror, so that the size and/or dimensions of the garment (110) can be recorded in at least two planes.

## Revendications

1. Procédé pour déterminer les dimensions d'au moins un pied d'un utilisateur, comprenant les étapes suivantes :
a) Placer au moins un pied d'un utilisateur sur un objet de référence aux dimensions connues, en utilisant comme objet de référence une feuille blanche au format standardisé,
b) photographier le pied ou le contour (12) du pied et l'objet de référence enregistré sur l'objet de référence avec un appareil photo numérique d'un terminal de téléphone mobile, par exemple un smartphone, dans lequel le pied ou le contour (12) et l'objet de référence sont représentés sur la même photographie,
c) calcul des dimensions du pied à partir de la photographie, sur la base des dimensions connues de l'objet de référence, au moyen d'un logiciel installé en tant qu'application sur le terminal mobile ou sur un serveur, les contours gênants qui sont visibles sur la photographie prise étant calculés, dans lequel
l'objet de référence et le pied sont situés dans un plan commun ou dans deux plans dont la distance et l'angle entre eux sont connus au moment de la photographie.

2. Procédé selon la revendication 1, dans lequel après la photographie selon l'étape b) du procédé et avant le calcul des dimensions selon l'étape c) du procédé, les étapes suivantes sont réalisées :
- Suppression de la distorsion de la perspective et/ou de la déformation de la photo capturée,
- Recherche des contours (10a) du pied ou du contour enregistré (12) du pied à l'aide d'un algorithme de recherche de contours, dans lequel en particulier les contours gênants dont la longueur est inférieure à une valeur seuil prédéterminée, en particulier inférieure à 5 mm, sont éliminés.

3. Procédé selon la revendication 2, dans lequel, pour trouver le contour enregistré (12) du pied, on utilise un algorithme de détection des bords comprenant les étapes suivantes :
- Créez un histogramme de valeur de tonalité de la distribution de la luminosité de la photo capturée,
- créer une image en noir et blanc à partir de la photographie capturée, dans laquelle chaque pixel de la photographie capturée ayant une luminosité inférieure à un seuil de luminosité présélectionné est défini comme étant noir et les pixels restants sont définis comme étant blancs, dans laquelle en particulier la médiane de l'histogramme des valeurs tonales de la distribution de luminosité est présélectionnée comme étant le seuil de luminosité.

4. Procédé selon l'une quelconque des revendications 1 à 3, dans lequel l'objet de référence est plat et a une forme rectangulaire, dans lequel un coin du rectangle qui n'est pas visible sur la photographie est calculé par l'intersection des extensions des bords adjacents, à condition que ce coin soit caché sur la photographie par une partie du corps de l'utilisateur ou un autre objet.

5. Procédé selon l'une quelconque des revendications 1 à 4, dans lequel, lors de la photographie directe du pied, la partie inférieure de la jambe de l'utilisateur (18) est extraite de la photographie par les étapes suivantes si elle est représentée sur la photographie :
- Détermination du contour commun du pied et de la jambe inférieure (18) par un algorithme de détection des bords,
- déterminer les deux points de transition (16a, 16b) où le contour du pied se fond dans le contour de la jambe inférieure (18), aux deux points où le contour commun du pied et de la jambe inférieure (18) a une flexion qui dépasse un seuil de flexion prédéterminé,
- Relier les deux points de transition (16a, 16b) pour créer un contour autonome (10a) du pied.

6. Procédé selon l'une quelconque des revendications 1 à 5, dans lequel, lors de la photographie du contour (12) enregistré du pied, le contour (12) est détecté par un algorithme de recherche de bord par les étapes suivantes, même si son point de départ (12a) et son point d'arrivée (12b) ne sont pas identiques et qu'il ne forme donc pas un contour (12) complètement fermé :
- déterminer la position du point de départ (12a) et du point d'extrémité (12b) du contour (12) par l'algorithme de recherche de bord, dans lequel un contour fermé (12) est supposé si la distance (12) entre le point de départ (12a) et le point d'extrémité (12b) est inférieure à un seuil de distance défini.

7. Procédé selon l'une quelconque des revendications 1 à 6, comprenant les étapes supplémentaires suivantes pour déterminer la taille et/ou les dimensions d'un vêtement (110):
a) création d'une image du vêtement (110) et de l'objet de référence (114) avec des dimensions connues, dans laquelle le vêtement (110) et l'objet de référence (114) sont imagés sur la même image et le vêtement (110) et l'objet de référence (114) sont disposés dans un plan commun ou dans deux plans dont la distance et l'angle l'un par rapport à l'autre sont connus pendant la photographie,
b) calculer les dimensions et/ou la taille du vêtement (110) à partir de l'image capturée sur la base des dimensions connues de l'objet de référence (114).

8. Procédé selon la revendication 7, dans lequel, en plus d'une représentation du vêtement (110) selon une perspective directe, au moins une autre représentation selon une autre perspective est enregistrée sur l'image du vêtement (110) en utilisant au moins un élément réflecteur (122a, 122b, 122c), en particulier un miroir, de sorte que la taille et/ou les dimensions du vêtement (110) peuvent être enregistrées dans au moins deux plans.
